# EUROPEAN PATENT SPECIFICATION

(11) **EP 3 302 319 B1**
(45) Date of publication and mention of the grant of the patent: **10.04.2019**
(21) Application number: 16727378.8
(22) Date of filing: 26.05.2016
(51) Int. Cl.: A61B 17/54

(54) **MICRODERMABRASION DEVICE WITH SKIN DOME MEASUREMENT TO ADJUST VACUUM SETTING**
MIKRODERMABRASIONSVORRRICHTUNG MIT HAUTKUPPELMESSUNG ZUR ANPASSUNG DER VAKUUMEINSTELLUNG
DISPOSITIF DE MICRODERMABRASION COMPRENANT UN SYSTÈME DE MESURE DE COURBURE DE LA PEAU POUR AJUSTER LE RÉGLAGE DU VIDE

(30) Priority: 03.06.2015 EP 15170592
(43) Date of publication of application: 11.04.2018
(73) Proprietor: Koninklijke Philips N.V., 5656 AE Eindhoven (NL)
(72) Inventor: BEIJENS, Linda, Goverdina, Maria, 5656 AE Eindhoven (NL); JURNA, Martin, 5656 AE Eindhoven (NL); JANSEN, Marjolein, Yvonne, 5656 AE Eindhoven (NL); PEETERS, Felix, Godfried, Peter, 5656 AE Eindhoven (NL)
(74) Representative: van Oudheusden-Perset, Laure E.
(86) International application number: PCT/EP2016/061954
(87) International publication number: WO 2016/193127

(56) References cited:
- WO-A1-2014/136013
- WO-A1-2014/191149
- WO-A1-2014/191263
- US-A1- 2006 189 964
- US-A1- 2008 200 778
- US-A1- 2008 249 537

## Description

### FIELD OF THE INVENTION

The invention relates to a microdermabrasion device and to a non-therapeutic method for the controlled removal of at least part of the stratum corneum with such device.

### BACKGROUND OF THE INVENTION

Microdermabrasion devices are known in the art. US 6,241,739 describes for instance a treatment tool and tissue collection system, for removal of outer layers of skin to provide a revitalized, fresh skin surface, and a method of using same, comprising an abrasive tipped tool mounted on the end of a tube, said tube being connected to a source of vacuum. The vacuum aids in maintaining intimate contact between the abrasive tip and the skin during the treatment process and transports the removed tissue to a collection container.

Especially, this document describes a device for removing portions of the outer layers of skin comprising a source of a vacuum, and a tube with an abrasive treatment tip thereon for dislodging cells from a surface being treated, the tube being attached to the source of vacuum so that a lumen through the tube has a reduced pressure therein which is less than the ambient pressure surrounding the tube, the abrasive treatment tip having at least one opening therein for applying the reduced pressure within the tube to a skin surface, said vacuum causing the skin being treated to have an increased area of contact with the abrasive tip, the vacuum also functioning to collect tissue or cells removed from the skin surface being treated. Further, this document describes a method of treating the skin surface of a patient to remove surface cells and reduce undesirable skin blemishes comprising providing a tubular treatment tool with an abrasive skin contacting surface, providing a pressure through a lumen within the tubular treatment tool which is less than ambient pressure surrounding the treatment tube, and bringing the abrasive skin contacting surface into contact with the skin surface to be treated while said lesser pressure is delivered to the skin surface through the lumen and moving the abrasive skin contacting surface across the skin surface.

US 2006/189964 describes methods and apparatuses to apply substances to a biological tissue. The tissue having a target below a surface is stretched to provide openings in a surface. Stretching forces a material from the target onto the surface. A first substance may be applied while stretching to promote cleaning the tissue. A second substance may be applied to the surface. Energy may be applied to the tissue. Then the tissue is relaxed to draw the second substance through the openings into the tissue. The tissue may be stretched by applying a negative pressure, and relaxed by removing the negative pressure. The first substance may be an abrasive material. The second substance is a medicine, a moisturizer, a lotion, or any combination thereof. The second substance may be applied to the biological tissue using a positive pressure. The apparatus to apply substances may be a handheld device.

US 2008/249537 describes a skin resurfacing device for peeling the outermost layer of the skin for renewing the skin surface and repairing skin damage including a housing, a skin sensor, and a skin treater. The skin treater consists of an abrasive tip, a first end, a transparent portion, a first filter, and a second end, all of which are detachable and replaceable. The skin treater is connected to a vacuum source by a tubular hose. The vacuum provides both closeness of contact between the abrasive tip and the user's skin and the suctioning of skin debris peeled off. The skin treater, especially the abrasive tip is made with common material and mass production process so that they are disposable and economic.

WO 2014/191263 describes a non-invasive device for skin rejuvenation using a treatment pressure below ambient pressure, and provides a method and a computer program product. The non-invasive device comprises a suction chamber having a skin contact surface comprising an opening for exposing the skin tissue to the suction chamber for applying a suction force to an outer surface of the skin tissue. The opening in the suction chamber is less than 10 square millimeters. The suction chamber is dimensioned so as to allow it to be manually applied to the outer surface of the skin tissue. The non-invasive device further comprises a controller for controlling a level of treatment pressure (Pt) inside the suction chamber and for controlling an application time interval (Δt) of the treatment pressure for damaging an interface between an epidermis layer and a dermis layer of the skin tissue.

US 2008/200778 describes that an adjustable massage apparatus has a massage effect which is based on the suction effect created in connection with the treatment head of the apparatus. The treatment head of the apparatus has a surface which comes into contact with the skin, a frame, and a low-pressure chamber, associated with the surface for creating a low-pressure suction in order to lift the skin. The apparatus has a sensor for measuring characteristics of skin tissue, and elements to automatically adjust the low-pressure suction to the desired value, on the basis of the obtained measurements. WO 2014/136013 describes a microdermabrasion device. The device has a suction path along which skin fragments removed by the device are drawn, and a detection unit configured to determine one or more characteristics of skin fragments drawn along the suction path. The detection unit is configured to determine the depth of abrasion performed by the device based on the one or more characteristics of skin fragments drawn along the suction path. This document also describes a method of determining the operating depth of abrasion of a microdermabrasion device).

WO 2014/191149 describes a microdermabrasion device comprising a vacuum system and a device tip, wherein the vacuum system comprises a channel with a channel inlet at the device tip, wherein the channel inlet is surrounded by a channel rim which facilitates gliding of the device tip over a skin, and wherein the device tip comprises a microdermabrasion zone configured remote from the channel inlet with a recession configured between the microdermabrasion zone and the channel rim.

### SUMMARY OF THE INVENTION

The "micro dermo abrasion" or "mircodermabrasion" (MDA) technique is being used to help the upper skin layer (the so called stratum corneum) to renew in a faster way than it would normally do. Traditionally, crystal microdermabrasion system contains a pump, a connecting tube, a hand piece, and a vacuum source. While the pump creates a highpressure stream of inert crystals, like aluminum oxide, to abrade the skin, the vacuum removes the crystals and exfoliated skin cells. Instead of abrasion with particles in a gas stream, also a roughened surface, such as a diamond surface, of the tip of the device can be used. This is for instance known as (diamond) microdermabrasion. Unlike the crystal microdermabrasion system, the (diamond) microdermabrasion does not produce particles from crystals that may be inhaled into patients' nose or blow into the eyes.

The present invention especially relates amongst others to a microdermabrasion device with a stationary abrasion zone (i.e. no moving abrasive part) and to microdermabrasion devices which use the abrasion zone as abrasive means, thus without the use of (a flow of) abrasive particles that (is) are used to abrade the skin and may be sucked by the vacuum. However, in other embodiments, the present invention also relates to a microdermabrasion device wherein the microdermabrasion area may be moving (i.e. not stationary) and/or wherein (a flow of) abrasive particles (is) are applied.

The effect of the mechanical action of the microdermabrasion under a pressure component on the skin may depend on the elastic properties of the skin. For instance, a too high underpressure can cause the skin to be extended too much resulting in side effects like bruising or blisters whereas a too low underpressure leads to a non-optimal mechanical massage. Furthermore, a too high underpressure may create a higher friction during movement which can negatively impact the device handling. Further, the underpressure or vacuum may affect the abrading effect and/or the massage effect. Yet further, the skin properties and thereby the skin dome height vary within and among individuals as well as with age. For example, among the different treatment areas in the face and décolleté, there are areas where the skin is thinner and looser (e.g. under the eyes) than in other areas. Using the same underpressure settings at all treatment locations may result in unpleasant and more severe treatments on those delicate areas. On the other hand, for areas where the skin is very stiff, like the forehead, the level of massage and abrasion could be enhanced. To reach the optimal mechanical massage and abrasion the optimal underpressure setting should be selected. Current available microdermabrasion devices do not offer a large variety in underpressure settings and determining the optimal setting is very difficult for a consumer.

Hence, it is an aspect of the invention to provide an alternative microdermabrasion device, which preferably further at least partly obviates one or more of above-described drawbacks. Further, it is an aspect of the invention to provide an alternative non-therapeutic method for the controlled removal of at least part of the stratum corneum, which preferably further at least partly obviates one or more of above-described drawbacks.

With the present invention a microdermabrasion device is provided where the underpressure may e.g. be adjusted in such an extent that e.g. the skin dome height and thereby the level of massage and abrasion is constant for all subjects or treatment locations (including the delicate ones). The device includes a sensor for measuring the effect of the underpressure on the skin that is related to the elastic properties of the skin. The measurement outcome may provide input for an automatic adjustment of the underpressure setting to provide a constant and optimal treatment effect. The measurement can be performed at a single or multiple times and on one or more body areas. It can also be a continuous measurement during movement of the device over the skin while automatically adjusting the underpressure to the treated skin. It can also be a periodic measurement during movement of the device over the skin while automatically adjusting the underpressure to the treated skin. It may also be a continuous measurement during use of the device while the device is not moving over the skin while automatically adjusting the underpressure to the treated skin when considered necessary, etc..

Hence, in a first aspect the invention provides a microdermabrasion device (herein also indicated as "device") comprising a vacuum system and a device tip ("tip"), wherein the vacuum system is in fluid communication with (a channel inlet at) an inlet zone of the device tip, and wherein the vacuum system is configured to apply a vacuum to the inlet zone, wherein the inlet zone further comprises a sensor configured to measure a skin parameter of a part of a skin in (protruding into) the inlet zone and to provide a corresponding sensor signal, wherein the device tip further comprises a microdermabrasion zone configured to abrade (during use (of the device, especially by moving the microdermabrasion device over the skin)) a part of said skin, and wherein the microdermabrasion device further comprises a control unit configured to control (during use) the vacuum as function of sensor signal information derived from the sensor signal and a predetermined relation between the sensor signal information and a vacuum setting. The microdermabrasion device is especially a handheld device, for use by an (adult) human.

With such device, the massage function (especially caused by moving the device over the skin (and in contact with the skin) in combination with the vacuum) and abrasion function (especially caused by the microdermabrasion zone, optionally in combination with moving the microdermabrasion device over the skin (and in contact with the skin)) may optimally be executed, also taking into account different types of skin, but even aspects like movement of the device or earlier treatments with the device. In this way, each type of skin and each treatment may be chosen with optimal vacuum conditions to lead to the best results, convenient to the user.

Here below, the device is described in more detail, often with reference to the device when being applied as microdermabrasion device to the skin of a human. However, the device and accompanying claims relate also to the device per se. Application or use of the device may include movement of the device over the skin (i.e. moving while physically being into contact with the skin, especially moving while a skin dome protrudes into the inlet zone), but application or use may also include a stationary use, i.e. the device is not moved over the skin (while staying physically into contact with the skin).

As indicated above, the sensor is especially configured to measure a skin parameter of a part of a skin in the inlet zone and to provide a corresponding sensor signal. During use, the part of the skin may be configured to the inlet zone of the microdermabrasion device. Due to the vacuum applied by the vacuum system, i.e. an underpressure at the inlet zone, the skin will be sucked into the inlet zone. The sensor may measure the skin just before the inlet zone and/or sucked into the inlet zone. The sensor may be configured to measure a skin parameter of a part of a skin in the inlet zone, i.e. skin protruding into the inlet zone. Hence, the sensor may also be configured to measure a skin parameter of a part of a skin in the inlet zone.

The term "skin parameter" may especially refer to a protrusion depth of the skin into the inlet zone. Alternatively or additionally, the skin parameter may also refer to one or more of color and roughness of the skin. Hence, the sensor is especially configured to measure one or more of protrusion depth of the skin in the inlet zone, the color of the skin (in the inlet zone), and the roughness of the skin (in the inlet zone). The term "skin parameter" may also refer to a plurality of different skin parameters. The term "sensor signal" may also relate to a plurality of sensor signals.

Further, the sensor signal(s) related to the skin parameter(s) measured may be stored (in (a memory of) the control unit (see further also below). The sensor signal may be stored as such or may be stored after processing of the signal. Both the sensor signal as well as the processed sensor signal are herein indicated as "sensor signal information". For instance, after processing the sensor signal, information like flexibility or elasticity of the skin, thickness of the skin, type of skin, skin zone, etc. etc. may be derived.

Based on the sensor signal information and the predetermined relation between the sensor signal information and a vacuum setting (predetermined vacuum setting), the control unit can maintain the vacuum or change the vacuum to the predetermined vacuum setting when the vacuum differs from the predetermined vacuum setting. The term "vacuum setting" may e.g. refer to one or more of numerical values for the predetermined underpressure or e.g. settings of the pump (and of the bypass system; see also below). For instance, the predetermined setting may define that a deeply protruding skin or a flexible skin or periorbital skin (all examples of possible sensor signal information) is subjected to less vacuum than less deeply protruding skin or inflexible skin or forehead skin. Further, the control unit may optionally also derive from the skin whether e.g. a mild or more severe abrasion may be necessary and/or whether a mild or severe massage is necessary. Dependent upon the construction of the microdermabrasion device (especially when vacuum and abrasion are coupled or decoupled) the control unit may select the vacuum corresponding to a milder or more sever abrasion.

Hence, in an embodiment the control unit is configured to determine from the sensor signal information of the type of skin and control the vacuum as function of the skin type. Here, the term "type of skin" may refer to e.g. a body zone, but e.g. also to age of the skin. The latter may e.g. be derived from a roughness of the skin. Further, the firmness of the skin may be derived from the height of the skin dome in the device. Hence, in a specific embodiment the control unit is configured to determine from the sensor signal information a body zone and control the vacuum as function of the body zone. For instance, the control unit may determine whether the skin is the skin of a cheek, of a forehead, of a periorbital area, of an underarm, a lower leg, etc. etc..

In a specific embodiment, it may be desirable to keep the massage effect substantially constant. Hence, in an embodiment the control unit is configured to maintain a constant protrusion of the skin into the inlet zone. Hence, the skin depth in the inlet zone may substantially be constant.

The sensor may be selected from the group consisting of an optical sensor and an electrical conductivity sensor. Hence, the sensor signal may be an optical sensor signal or e.g. an electrical conductivity sensor signal. Also combinations of two or more different types of sensor may be used. The optical sensor is especially configured to measure optical phenomena, such as emission, reflection, transmission the electrical conductivity sensor is especially configured to measure electrical conductivity (or electrical resistivity). The sensor may measure in one or more ways. Further, the term "sensor" may also refer to a plurality of (different) sensors. Especially, the sensor(s) may be configured to measure one or more of protrusion depth of the skin in the inlet zone, the color of the skin, and the roughness of the skin. The sensor may measure once, may measure continuously, may measure periodically, etc.. The sensor may measure at one position or may measure at a plurality of positions. Hence, in embodiments the control unit and the sensor are configured to apply an optical measurement including one or more from the group consisting of (i) chromatic aberration, (ii) triangulation, (iii) light transmission, and (iv) light reflection, and wherein the sensor is especially configured to measure one or more of protrusion depth of the skin in the inlet zone, the color of the skin, and the roughness of the skin.

The control of the vacuum or underpressure to the inlet zone may be executed in several ways. In an embodiment, the control unit changes the vacuum provided by a pump (directly). For instance, the power supply to the pump may be controlled. Additionally or alternatively, one may keep e.g. the pump at a constant level, but control a leakage of the vacuum. A larger leakage reduces the vacuum or underpressure and a smaller leakage or no leakage increase the vacuum and (thus) reduces the pressure (larger underpressure). Hence, in an embodiment the vacuum system comprises a pump and a bypass system with a controllable vacuum leakage (from the inlet zone (to ambient)), wherein the control unit is configured to control the vacuum applied to the inlet zone by controlling the controllable vacuum leakage. Hence, the vacuum system is in fluid contact with the inlet zone, and the bypass system is also in fluid contact with the inlet zone. The bypass system may e.g. include a further channel with a controllable channel thickness (at one or more locations in the channel).For instance, the bypass system may include one or more valves for controlling the vacuum leakage. With the bypass system, it may be possible to keep the pump pumping stationary and nevertheless tuning the vacuum. For controlling the vacuum (to the inlet zone) with the vacuum system comprising the bypass system, the bypass system may include one or more of a single valve (rotating) or double valve (rotating), a non-symmetrical rotating element to control the vacuum leakage, etc.. The valve may include a mechanical valve, a magnetic valve, a pressure controlled valve (pneumatically controlled), a rotating disk with holes, which hole may vary in dimensions and/or the wheel rotation speed may be varied, etc. etc.. The bypass system may thus provide a controlled vacuum leakage due to a fluid contact with ambient (especially other than the inlet zone). Therefore, in embodiments the vacuum may essentially only controlled via the bypass system. This may also allow a relatively simple pump.

The vacuum system comprises a channel with a channel inlet (or "channel opening") at an inlet zone. The channel is in fluid connection with the pump (for creating the vacuum) and the inlet zone. Further, the channel may be in fluid communication with the (optional) bypass system. The vacuum system is thus especially controllable in the sense that the underpressure may be adaptable at a plurality of pressures (see also below for pressures), such as e.g. a stepwise controllable underpressure.

The vacuum system may comprise a source of vacuum, such as pump, configured to provide a suction flow in a direction from the channel inlet to the source of vacuum, such as a pump. The channel inlet or inlet zone, which is at the device tip, is thus configured upstream of the vacuum pump. Especially, the device may be configured to provide a negative pressure ("underpressure") in the range of 5-80 kPa, such as especially 15-60 kPa, such as in the range of 20-40 kPa. This may especially imply that when the skin is in contact with the inlet zone, and closes off the inlet zone, the device is able to provide a pressure which is in the range of 15-60 kPa lower than atmospheric pressure. Hence, the term underpressure may especially indicate that when the skin is in contact with the inlet zone, the skin may be sucked at least partly into the inlet zone due to the suction of the vacuum system, leading to an underpressure in the inlet zone relative to ambient pressure. Hence, especially the vacuum system is configured to suck gas from the inlet zone away into the vacuum system.

In general, the inlet zone is configured in such a way, that a good closing connection with the skin may be achieved. Especially, the inlet zone comprises a rim, herein also indicated as "channel rim". This channel rim may be a (slightly) protruding part of the device tip. The channel rim may also be seen as a distal part or end part of the channel opening. Especially this rim will be in contact with the skin of a user during use of the device. Optionally, this rim may comprise the microdermabrasion area with abrading material (see also below).

Further, it may be desirable that the user may also influence the settings of the microdermabrasion device. For instance, the user may choose between e.g. options "light" and "intense", or "light", "medium", and "intense", or "high", "medium", and "low", etc., or the user interface may be configured to allow indicating the body zone that is to be treated, or the user interface may be configured to allow the user selecting a specific program (e.g. face & arms), etc. etc.. The user interface may optionally comprise a graphical user interface. Further, optionally the user interface is a remote interface, such as an App on a smart phone, iPhone, tablet or other (portable) electronic device. With providing information via the user interface, the control unit may impose boundaries to the possibilities that may be chosen by the control unit when providing instructions to the vacuum system. For instance, the range of the vacuum setting may be limited and only a sub selection may be allowed (e.g. the stronger vacuums when choosing "intense" or "high"). Hence, in an embodiment the microdermabrasion device further comprises a user interface configured to allow a user select a user input parameter related to a strength of the vacuum, and wherein the control unit is configured to control the vacuum (also) as function of the user input parameter.

The microdermabrasion device may also be configured to determine from the sensor signal motion of the microdermabrasion device. Additionally or alternatively, the microdermabrasion device may include a motion sensor. When the microdermabrasion device can determine motion, also vacuum may automatically adapted, as e.g. the microdermabrasion device may recognize a movement to a more flexible or less flexible skin zone. Hence, the microdermabrasion device may anticipate the vacuum settings to be. Hence, in yet a further embodiment, the microdermabrasion device may further optionally comprise a motion sensor, wherein the control unit is configured to determine from one or more of sensor signal information derived from the sensor signal and motion sensor signal information derived from a motion sensor signal from the motion sensor one or more of a translation speed parameter and a translation direction parameter of the microdermabrasion device (during use) and control the vacuum as function of one or more of said translation speed parameter and said translation direction parameter. The term "sensor signal information" refers to the sensor signal or information that is derived from such sensor signal. Likewise, the term "motion sensor signal information" refers to the sensor signal or information that is derived from such motion sensor signal. The term "sensor signal" refers especially to the sensor signal from an optical sensor and/or an electrical conductivity sensor.

In yet a further embodiment, the control unit is further configured to control the vacuum as function of one or more of a desired abrasion or effected abrasion (i.e. achieved abrasion). For instance, the microdermabrasion device may be configured to induce a stronger abrasive effect with a larger vacuum (although herein also embodiments are described wherein the massage effect and abrasive effect are decoupled). Hence, when the control unit determines from a sensor signal that a certain abrasion is needed, e.g. also on the bases of a user input, or determines that less abrasion is need, e.g. because of the fact that a certain zone has been abraded for a certain time, the vacuum may be decreased (i.e. less underpressure), such that abrasion may decrease.

The control unit may include a temporary memory. Optionally, the control unit also comprises a (remote) permanent memory for storage of sensor signal information. In this way, e.g. the control unit may generate a map of at least part of a human skin. In this way, it is also possible to derive information on skin (treatment) progress, or deterioration, on the basis of which the control unit may further adapt the vacuum setting. Further, the control unit may store treatment information, such as the vacuum conditions and time used for the treatment. Based thereon, the control unit may be configured to suggest further treatment. For instance, the microdermabrasion device may give instruction to stop treating a specific zone or to continue treatment of a specific zone. Such information may be provided on a display and/or may be provided via a sound signal and/or may be provided via a vibration signal. The display and (graphical) user interface may be integrated in a single unit. Hence, sensor signal information, as well as development over time, and conclusions thereon, may e.g. be displayed on a display of the sensor device (or with the App; see also above). Hence, in yet a further embodiment the control unit is configured to store one or more of (a) sensor signal information, and (b) treatment information, and the control unit is further configured to execute one or more of (i) controlling the vacuum as function of one or more of said sensor signal information and treatment information, and (ii) providing on a display information retrieved from one or more of said sensor signal information and treatment information.

There are a number of types of microdermabrasion devices.

For instance, abrasive particles may be used that are propelled by a gas flow to the skin (in or in front of the inlet zone) and thereby abrade the skin, or the microdermabrasion device comprises an area with abrasive material, which has an abrasive function when the microdermabrasion device is moved over the skin. In the former embodiment, the abrasive material comprises abrasive particles, which are provided in a flow, and in the latter embodiment, the abrasive material is immobilized, and e.g. comprised by a rim.

Optionally, the microdermabrasion device may also include a moving, such as rotating, element including abrasive material (especially a moving microdermabrasion zone).In such embodiment, the abrasive material is also immobilized, and may e.g. comprised by a movable element, such as a rotatable rim. In embodiments, the terms "rotation" or "rotating" may optionally also refer to "oscillation" or "oscillating". Hence, in embodiments the term "rotation" and similar terms refer to full rotations, and in other embodiments the term "rotation" and similar terms refer to rotations less than 360° among a rotational axis.

Of course, also combinations of two or more embodiments may be applied.

In principle, for all these embodiments the invention may be useful, as in all cases a vacuum may be used to remove the material that is abraded, and optionally also for massage properties. Hence, in a specific embodiment the microdermabrasion device further comprises an abrading material system configured to provide in a gas flow abrading material to the microdermabrasion zone. The terms "abrasive material" and "abrading material" may substantially refer to the same material; particle properties of embodiments of such materials are defined below (see e.g. information concerning "particulate material"). Alternatively, (or optionally additionally), the microdermabrasion zone (of the microdermabrasion device) comprises a microdermabrasion area comprising immobilized abrading material.

It has been found that by a specific geometry the gliding force can be reduced and/or the treatment area can be increased with the same gliding force. Especially, in embodiments the invention provides two zones, especially two rings, of which one zone provides the abrasive function and of which one zone provides a gliding function (to facilitate gliding over the skin). For instance, two zones, especially two rings, may be configured around the vacuum opening. The inner zone, especially the ring, around the vacuum opening is the gliding zone (or especially the gliding ring). It has substantially no abrasion / abrasive function and may be smooth. Hence, this zone, especially the ring, facilitates gliding of the device tip over a skin (of a human). The outer zone, especially the ring, is the abrasive zone or area. Hence, this zone, especially the ring, may have abrasive roughness. The roughness and sharpness of the abrasive texture may depend on the treatment goal and personal preferences of users. Hence, this zone, especially the ring, is configured to provide the abrasive function for the controlled removal of at least part of the stratum corneum. In between the two zones, especially in between the rings, there is an area that is further away from the skin. This area separates the two zones, especially the rings. The invention is not limited to ring-like microdermabrasion areas and/or ring-like channel rims (or gliding zones).

The gliding zone, especially the ring (especially the inner ring), may be needed for easy gliding over the skin, which will make the device easy to use. Friction can be tuned by (ring) shape, material roughness, material type, surface topography, coatings and other friction reduction methods. Lowering vacuum level also helps reducing friction, but this may be less desired because this limits the benefits (see also above). The outer zone, especially the (outer) ring, contains the abrasive texture. Abrasiveness can be tuned by texture sharpness, texture roughness, material type, ring shape, ring height, etc.. The area in between the zones, especially the rings, which is further away from the skin, has an important function. It separates the gliding area from the abrasive area. It may further gives pressure to the gliding zone which may need pressure to be able to close the vacuum opening on the skin.

In yet a further aspect, the invention also provides a non-therapeutic method for the controlled removal of at least part of the stratum corneum, the method comprising contacting the microdermabrasion device, such as defined herein, with part of a skin and removing at least part of the stratum corneum while applying a vacuum to the inlet zone.

Especially, the channel opening (with its channel rim) is configured such that a suitable vacuum area is obtained. The channel opening, especially the channel rim has (or provides) a vacuum area in the range of 10-400 mm², such as 10-200 mm², such as at least 20 mm², like especially at least 45 mm², especially in the range of 45-75 mm², such as especially 50-75 mm². This vacuum area is especially the area enclosed by the channel rim. It is especially also the (cross-sectional) area of the inlet zone.

As indicated above, the abrasion zone has abrasive properties, such as due to microscopic structures that facilitate abrasion of the upper part of the skin. Such microscopic structures may for instance be selected from the group consisting of alumina structures, such as particles, and diamond structures, such as diamond particles. These structures are comprised by the abrasion rim, i.e. are attached or part of the rim. Especially, the microdermabrasion area comprises abrasive structures, such as particulate material, attached to the microdermabrasion area having mean dimensions in the range of 1-1000 µm, such as 2-300 µm, like 5-80 µm or 120-200 µm. These dimensions may also apply when a gas flow with abrading particles is applied. Alternatively or additionally, the microscopic structures may for instance be selected from the group consisting of silicon carbide structures, such as silicon carbide particles, and metal nitride structures, such as metal nitride particles. Alternatively or additionally, the microscopic structures may for instance be selected from the group consisting of metal oxide structures, such as aluminum oxide particles and aluminum oxide structures. Further options of microscopic structures may for instance be selected from the group consisting of diamond structures, boron nitride structures, silicon carbide structures (see also above), glass beads, steel grit structures, other metal grit structures, zirconium oxide structures, and quartz structures. Combinations of different kind of structures, both in chemical composition and/or dimensions, may also be applied. In an embodiment, the quotient of the number of abrasive particles at the channel rim is especially 10% or less, more especially 5% or less, even more especially 1% or less of the number of abrasive particles comprised by the microdermabrasion area, especially 0.1% or less. In an embodiment, such abrasive particles are not comprised at all by the channel rim. The lower content or absence of such particles by the channel rim may facilitate gliding. The numbers given here as especially provided as an indication for certain embodiments to indicate the difference between the functionality of the channel rim and the microdermabrasion area.

The abrasive particles are especially available in the microdermabrasion area in a density in the range of 20-500 particles/mm². Especially particles in the size of 2-200 µm are available in this density (either mobile or immobilized in a microdermabrasion area). As indicated above, the channel ring or gliding, and the optional second gliding zone (see further below), have substantially no abrasive particles, or, as indicated above, especially 10% or less, more especially 5% or less, even more especially 1% or less of the number of abrasive particles comprised by the microdermabrasion area. Hence, assuming e.g. the microdermabrasion area having 250 particles of with one or more dimensions in the range of 100 µm per mm2, the number of such particles at the gliding zone(s) may be in the range of 25/mm² or lower (respectively). The abrasive particles may e.g. be glued to a surface, to provide the microdermabrasion area. However, alternative options are also possible. An abrasive surface can be made in many ways. Abrasive particles may be glued or metal plated. An abrasive structure can also be made from a solid material by machining or sanding a material. A surface treatment by a laser is also possible. Also by inject molding an abrasive surface can be created.

In an embodiment, the microdermabrasion area is stationary, i.e. especially the microdermabrasion area is not configured to move relative to the device. In yet another embodiment, the microdermabrasion area may be able to move. For instance, the device may be configured to let the microdermabrasion area vibrate. Optionally or additionally, the device may be configured to let the microdermabrasion area rotate. Such rotation may also include a vibration movement, for instance when the rotation is only a small rotation hence and forth (see also above when describing "oscillation").

With such MDA device, at least part of the stratum corneum can be removed from the skin of a human. This can be done in a non-therapeutical treatment, such as a cosmetic treatment. Hence, the invention also provides a non-therapeutic method for the controlled removal of at least part of the stratum corneum, the method comprising contacting the microdermabrasion device as defined herein with part of a skin and removing at least part of the stratum corneum while applying a vacuum to the vacuum channel. An advantage of the method and device of the invention is also that it (they) can be used for reducing a lateral force (gliding force) that has to be applied by a user when removing at least part of the stratum corneum with said device. Alternatively or additionally, the method and/or device as described herein can be used for reducing lateral force (gliding force) when removing at least part of the stratum corneum with said device. Further, alternatively or additionally, the method and/or device as described herein can be used for reducing a lateral force that has to be applied by a user when removing at least part of the stratum corneum with said device. Alternatively or additionally, the method and/or device as described herein can be used for increasing the treatment efficiency while not substantially increasing the vacuum level, keeping the same vacuum level, or even reducing, the vacuum level. Alternatively or additionally, the method and/or device as described herein can be used for increasing the treatment are while not substantially increasing, or even reducing, the gliding force.

Further variants and embodiments described in WO2014191149 may also be used in combination with the present invention.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Figs. 1a-1e schematically depict some aspects of the MDA device;
Figs. 2a-2c schematically depict some measurements embodiments;
Figs. 3a-3d schematically depict some possible embodiment of the bypass system; and
Figs. 4a-4b schematically depict some aspects of the control of the device.

The schematic drawings are not necessarily on scale.

### DETAILED DESCRIPTION OF THE EMBODIMENTS

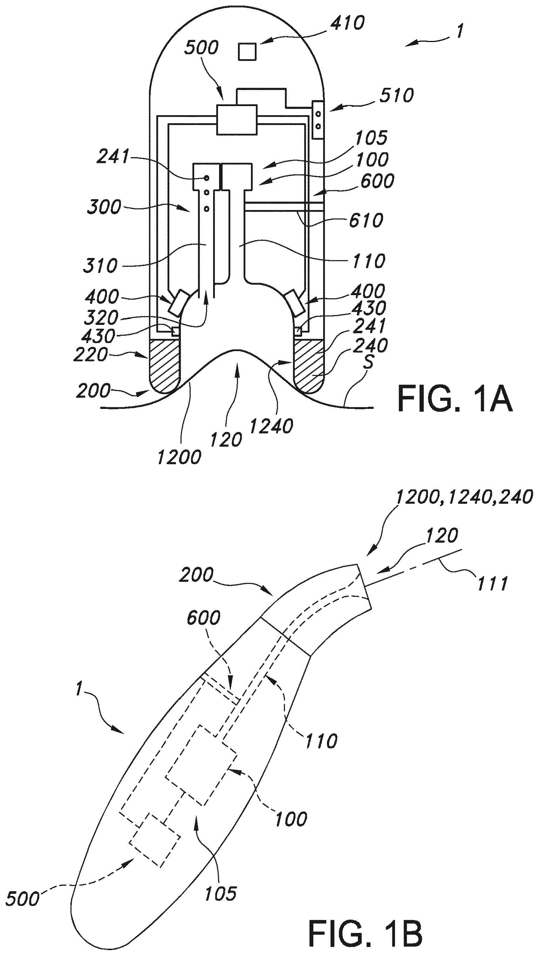

Fig. 1a schematically depicts an embodiment of the microdermabrasion device 1 including optional variants that may be included or of which some may and others may not be included dependent upon the specific embodiment desired. Fig. 1a shows microdermabrasion device 1 comprising a vacuum system 100 and a device tip 200. Here, the vacuum system 100 comprises a channel 110 with a channel inlet 120 at an inlet zone 1200 of the device tip 200. The vacuum system 100 is configured to apply a vacuum to the inlet zone 1200. To this end, the vacuum system 100 also comprises a pump 105. The channel opening, may provide a vacuum area in the range of 10-400 mm², such as at least 45 mm², especially in the range of 45-400 mm². Further, the inlet zone 1200 comprises a sensor 400 configured to measure a skin parameter of a part of a skin in the inlet zone 1200 and to provide a corresponding sensor signal. The skin is indicated as line with reference S, and is of course not a part of the microdermabrasion device 1. Here, by way of example four sensors are schematically depicted. These sensors may e.g. be optical sensors. These sensors may also be configured to measure e.g. electrical conductivity (of the skin), which changes when e.g. an electrode is in physical contact with the skin. Hence, the items indicated with reference 400 may e.g. also refer to sets of electrodes (e.g. indicated with reference 430) between which resistance or conductivity is measured, and wherein the device, 1, such as the control unit 500, may apply a potential difference between.

The device tip 200 further comprises a microdermabrasion zone 1240 configured to abrade - during use - a part of said skin. Here, two variants are depicted which are in general not combined, but which are combined for the sake of economy in this drawing. Here, the microdermabrasion device 1 further comprises an abrading material system 300 configured to provide in a gas flow abrading material 241 (such as the above defined particles / particulate material) to the microdermabrasion zone 1240. To this end, the abrading material system 200 may comprise a channel 310 for providing the abrading material to the inlet zone, with outlet 320 in this inlet zone 1200. The particles hit the skin S and thereby abrade the skin S. However, here also the variant is depicted wherein the microdermabrasion zone 1240 comprises a microdermabrasion area 240 comprising immobilized abrading material 241. The microdermabrasion zone 1240 and the inlet zone 1200 may at least partially coincide.

Reference 510 indicates a user interface, configured to allow a user select a user input parameter related to a strength of the vacuum. The control unit 500 may be configured to control the vacuum as function of the user input parameter.

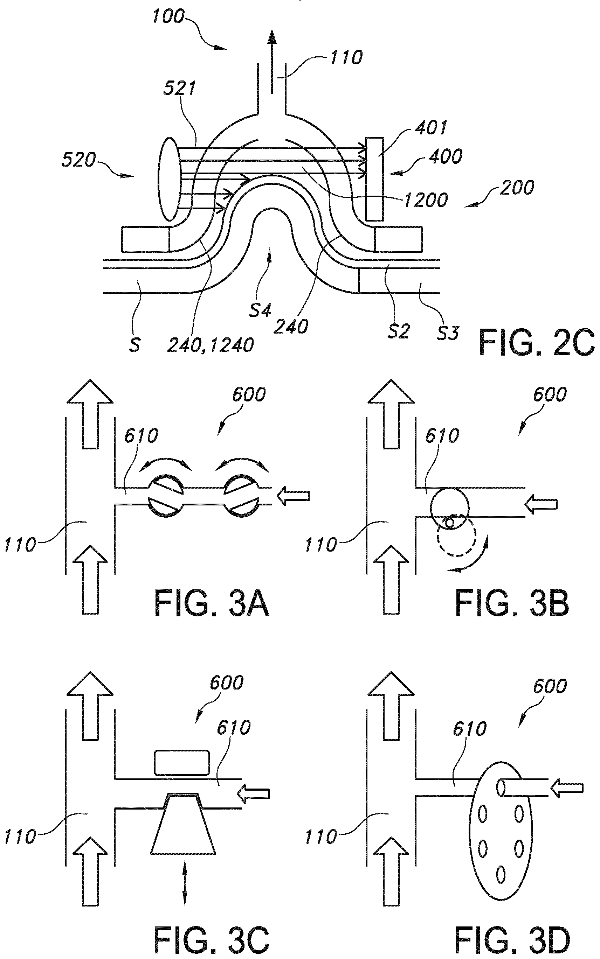

Further, the microdermabrasion device of Fig. 1a is depicted with a bypass system 600 with a controllable vacuum leakage (here including a valve). Reference 610 is a channel through which vacuum may leak (i.e. air may be introduced (from ambient) into the inlet zone 1200), see further also Figs. 3a-3d. The control unit 500 may be configured to control the vacuum applied to the inlet zone by controlling the controllable vacuum leakage.

Reference 410 indicates an optional motion sensor. The control unit 500 may be configured to determine from one or more sensor signal information, derived from the sensor signal, and motion sensor signal information, derived from a motion sensor signal from the motion sensor 410, one or more of a translation speed parameter and a translation direction parameter of the microdermabrasion device 1 (during use) and control the vacuum as function of one or more of said translation speed parameter and said translation direction parameter.

Fig. 1b schematically depicts an embodiment of a microdermabrasion device 1. This device 1 comprises a vacuum system 100, with a pump 105 and a channel 110. Further, this device 1 comprises a device tip 200. Pump 105 can suck air into the channel 110. Channel 110 has a channel inlet 120 at the device tip 200. In other words, the device tip has a channel inlet 120 which is part of the channel 110 of the vacuum system 100. The device tip 200 further comprises a microdermabrasion zone 1240 with a microdermabrasion area 240.

Reference 111 indicates a (virtual) channel axis. The microdermabrasion area 240 may include abrasive structures (not depicted), which are known per se (see also above).

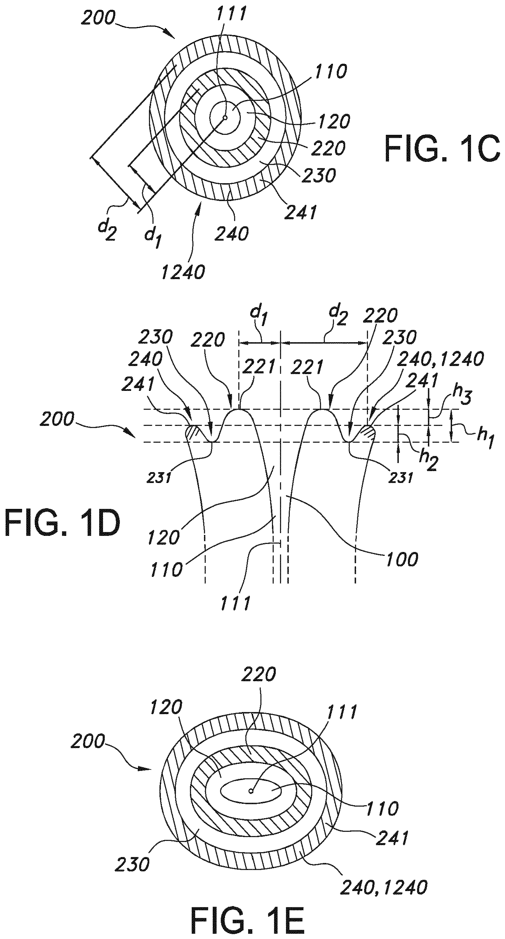

Fig. 1c schematically shows a top view of an embodiment of the microdermabrasion device. The distance from the (top of the) channel rim 220 to the channel axis 111 is indicated with d1, and the distance from the (top of the) abrasion zone 240 is indicated with d2, with d2>d1.

Fig. 1d schematically depicts a cross-sectional view of an embodiment of the microdermabrasion device. Here, wherein the channel rim 220 has relative to the recession bottom 231 a channel rim height h1. Further, the microdermabrasion area 240 has relative to the recession bottom 231 a microdermabrasion area height h2. For instance, the channel rim height h1 and the microdermabrasion area height h2 have a height difference h3 in the range of 0-5 mm. Note that both the channel rim 220 (or gliding zone) and abrasion zone 240 in Fig. 1d are schematically depicted as having a curvature in a direction of the channel axis. Such curved surfaces may be comfortable when performing the microdermabrasion method. The top of the rim 220 is indicated with reference 221; the top of the abrasion zone is indicated with reference 241. The channel inlet 120 is (perimetrically) surrounded by a channel rim 220. This channel rim 220 may facilitate gliding of the device tip 200 over a skin (not shown). The microdermabrasion area is 240 configured remote from the channel inlet 120 with a recession 230 configured between the microdermabrasion area 240 and the channel rim 220.

The present invention is not limited to handheld devices but may also relate to split devices, i.e. for instance a device with a main part, especially for providing the vacuum, and a tube with an abrasive treatment part, that can be moved at least partly independent of the main part. The tube may be a flexible tube.

Fig. 1e schematically depicts, together with Fig. 1c, some possible variants of the channel rim 220 and abrasion zone 240, varying from circular zones (1c) to elliptical zones (Ie), but also a circular channel rim but non-entirely (perimetrically) surrounding abrasion zone may be possible.

Referring to Figs. 1c, 1d, and 1e, the microdermabrasion area 240 may in embodiments be fixed (stationary). In such embodiments, the microdermabrasion area 240 may not (be able to) move parallel or perpendicular to the channel axis 111. However, in other embodiments, the microdermabrasion area 240 may be able to vibrate or rotate. For instance, the microdermabrasion area 240 may vibrate in a direction (substantially) parallel to the channel axis. In embodiments, the height difference h3 may thus vary during use due to vibration. Other type of vibrations may also be possible. In embodiments, the microdermabrasion area 240 may also be able to rotate, e.g. around the channel axis 111. In embodiments, the height difference h3 may be adjustable by the user.

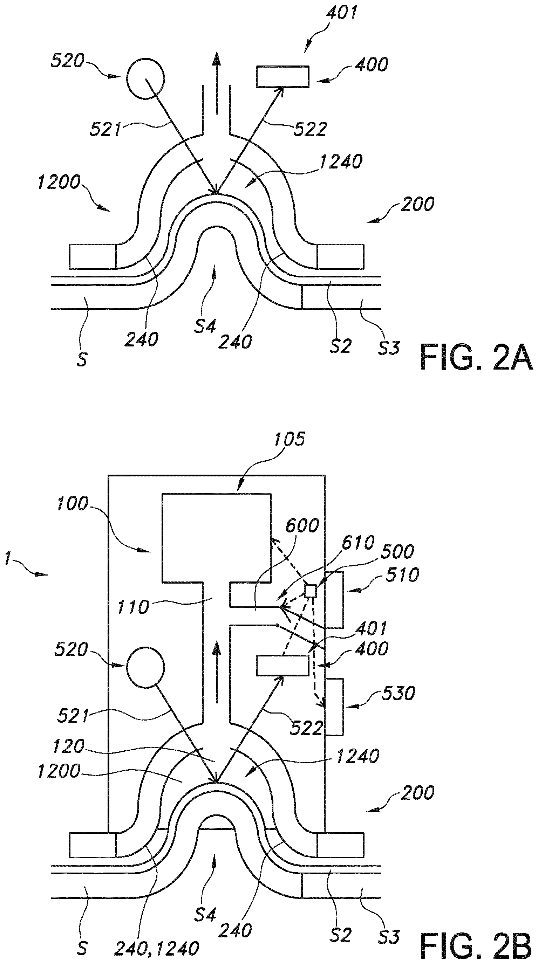

Figs. 2a-2c schematically depict some measurement embodiments. Fig. 2a shows an embodiment wherein e.g. a triangulation measurement is used (diagonal measurement). Skin references S2 and S3 indicate the epidermis and dermis, respectively. Reference S4 indicates the skin dome, protruding into the inlet zone 1200 due to the vacuum (indicated with the vertical arrow). References 520 and 401 indicate a light source and an optical sensor, respectively. The light source generates light beam 521 in the direction of the skin. Light reflected by the skin, indicated with reference 522, is measured by the optical sensor 401. Fig. 2a in principle thus also shows a setup for measuring one or more emission of the skin and reflectivity of the skin. Note that the light source 520 may be considered part of the sensor 400, as the light source 520 and optical sensor 401 are together configured as sensor 400, which is here used for e.g. a triangulation measurement.

Fig. 2b schematically depicts substantially the same embodiment as schematically depicted in Fig. 2a, but now with some further elements, including the vacuum system 100 and the bypass system 600. The control unit 500 may be used to control the bypass system 600 and vacuum system 100, as indicated by the dashed arrows. Further, the control unit 500 may display information on a display 530. Hence, in an embodiment of the invention comprises a microdermabrasion treatment tip to generate an underpressure on the skin. The underpressure will create a skin dome in the treatment tip by sealing off the skin at the edges of the treatment tip. A light source will be positioned at an angle of the skin dome and its light beam will be directed to the top of the skin dome and reflected to a light collector. For this purpose the treatment tip will be transparent to the wavelength. The angularity of the light reflected in the collector will determine the height of the skin dome. The information on skin dome height or related optimal vacuum setting can be displayed on the device housing or can be used to tune the underpressure level; either by tuning the vacuum pump or by changing a controlled leakage. The light source and collector could also be nested in the treatment tip.

Fig. 2c schematically depicts an embodiment wherein transmission is used. Hence, the sensor 400 here comprises a sensor configured to measure transmission (using a light source 520 and an optical sensor 401). This is a relative simple option, especially directed to measuring the skin dome height or skin protrusion factor. Of course, this method may be combined with other optical measurements, such as described above.

The invention may use amongst others the measurement of the skin dome height to provide feedback on the optimal underpressure setting and to use this feedback for automatic or manual underpressure selection. A high underpressure will result in a larger skin dome, thus more intense treatment effect and vice versa. This invention may lead to a controllable skin dome height independent from the mechanical properties of the skin leading to a controllable mechanical massage force and even abrasion of the skin.

For measurement of the skin dome height known measurement systems can be implemented in the treatment device or tip like chromatic aberration (vertical measurement), triangulation (diagonal measurement), light transmission (horizontal measurement), etc..

Measurement values may be checked by software against the skin dome height calibration constant for the selected setting. If the actual skin dome height deviates more than a predefined percentage the underpressure may be changed in one or both of the following ways: (a) tuning the vacuum pump motor, and (b) controlling the amount of air leakage from the closed vacuum system.

In an embodiment, a measurement mode is selected resulting in a single static (single position) or multiple static measurements. A numerical score or light color indicates the skin dome height and user is instructed to manually select the corresponding underpressure setting from an instruction. Especially, at least the underpressure is automatically selected based on the measurement(s) outcome.

The measurement may be done in parallel to the treatment in a continuous mode resulting in a facial map of skin dome heights and a resulting ideal setting or multiple settings for different body areas. Further, the measurement is done in parallel to the treatment in a continuous mode and underpressure setting is continuously adjusted to the measured skin dome height. The measurement of the (initial) skin dome can be saved into the device and displayed such to show progress of several skin properties related to the skin dome (e.g. elasticity) to indicate to the user improvements.

Further, the device could furthermore be equipped with a motion sensor to distinguish between a continuous and spot measurement of the skin dome. It could be set such that when the device is not moved during the treatment (located at turning points) the pressure will be released so the consumer can easily release the system from the skin (current problem indicated by consumers).

In an exemplary method embodiment, the following steps may be executed:
Step 1: user selects treatment setting (e.g. high, medium, low).
Step 2: device is placed onto skin treatment area.
Step 3: device is turned on, and measures at one or multiple fixed underpressure settings the skin dome height
Step 4: based on selected treatment setting (e.g. high, medium, low) the right level of underpressure is applied into the device for the rest of the treatment.

In another exemplary method embodiment, the following steps may be executed:
Step 1: User selects treatment setting (e.g. high, medium, low).
Step 2: Device is placed onto skin treatment area.
Step 3: Device is turned on, during the movement of the device the skin dome is kept at the same level as selected by the treatment level to obtain a uniform skin treatment.

Figs. 3a-3d schematically depicts several variants of the bypass system. The figures show different embodiments that provides a tuned level of underpressure at skin. Option A shows a single or double valve (rotating) to control the vacuum leakage. Option B shows a non-symmetrical rotating element to control the vacuum leakage. Option C shows a valve to control vacuum leakage. Such valve may e.g. a mechanical valve, a magnetic valve, a pressure controlled valve (pneumatically controlled), etc.. Option D shows a rotating disk with holes, which hole may vary in dimensions and/or the wheel rotation speed may be varied. Vacuum leaks to ambient as air may be sucked into the vacuum system (via the bypass system), as shown with the horizontal arrow.

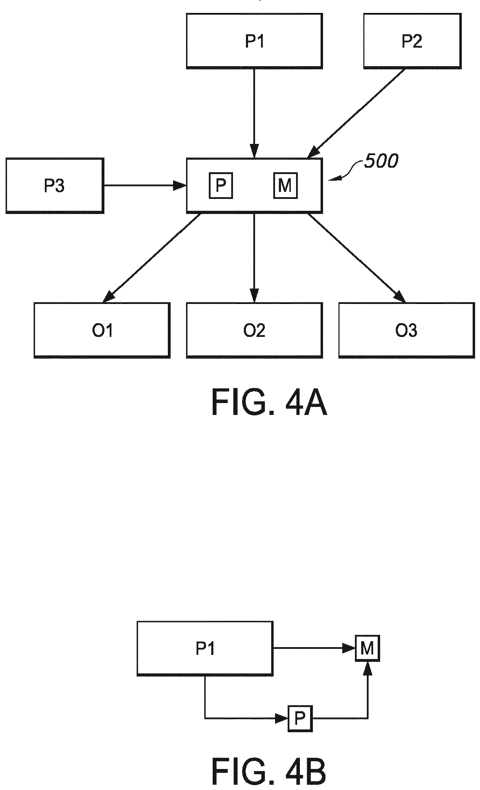

Fig. 4a schematically depict some information/data stream. The sensor signal PI and optionally a motion signal P2 are received by the control unit 500. Also information P3 provided by a user interface may be collected by the control unit 500. This information is processed into instructions to with respect to the vacuum O2 based on predefined setting, which may be stored in the memory M (or optionally a remote memory). Further, optionally information may be displayed O1 on a display. When an abrading material system O3 (see also ref. 300 in Fig. 1a) is available, also this system may be controlled. Reference P refers to a processor.

Note that substantially all aspects that may influence the vacuum settings may also influence the abrading material system settings, if such system is available.

Fig. 4b shows that sensor signal PI may be directly stored in the memory, but may also be first processed, to provide data on a higher level (meta data), and then be stored in the memory. Here the memory may be a temporary or permanent memory. The data may be sensor signal data, whereas the information stores may be these raw data or processed data, such as type of skin, flexibility of the skin, etc..

During use of the device, the device, more especially the vacuum system, especially applies a vacuum to the inlet zone. Hence, especially the device, more especially the vacuum system, is especially adapted to apply a vacuum to the inlet zo

During use of the device, the device, more especially the sensor especially measures a skin parameter of a part of the skin in the inlet zone, and provides a corresponding sensor signal. Hence, especially the device, more especially the sensor, is especially adapted to measure a skin parameter of a part of the skin in the inlet zone, and to provide a corresponding sensor signal.

During use of the device, the microdermabrasion zone moves, due to the manual movement of the device (by the user of the device) over the skin and/or due to the device rotating and/or vibrating such microdermabrasion zone, and the device thereby abrades a part of the skin. Hence, especially the device is adapted to abrade a part of said skin (due to said movement).

During use of the device, the device, more especially the control unit controls the vacuum as function of sensor signal information derived from the sensor signal and a predetermined relation between the sensor signal information and a vacuum setting (for the vacuum system (including the optional bypass system)). During use, the control unit may process (with a processor) the sensor signal into the sensor signal information. Hence, especially the device, more especially the control unit, is especially adapted to control the vacuum as function of sensor signal information derived from the sensor signal and a predetermined relation between the sensor signal information and a vacuum setting. Yet further, especially the device, more especially the control unit, is especially adapted to process (with a processor) the sensor signal into the sensor signal information.

The terms "upstream" and "downstream" relate to an arrangement of items or features relative to the propagation of the fluid, with in general upstream being at a higher pressure and downstream being at a lower pressure.

The term "substantially" herein, such as in "substantially consists", will be understood by the person skilled in the art. The term "substantially" may also include embodiments with "entirely", "completely", "all", etc. Hence, in embodiments the adjective substantially may also be removed. Where applicable, the term "substantially" may also relate to 90% or higher, such as 95% or higher, especially 99% or higher, even more especially 99.5% or higher, including 100%. The term "comprise" includes also embodiments wherein the term "comprises" means "consists of'. The term "and/or" especially the relates to one or more of the items mentioned before and after "and/or". For instance, a phrase "item 1 and/or item 2" and similar phrases may relate to one or more of item 1 and item 2. The term "comprising" may in an embodiment refer to "consisting of' but may in another embodiment also refer to "containing at least the defined species and optionally one or more other species".
Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices herein are amongst others described during operation. As will be clear to the person skilled in the art, the invention is not limited to methods of operation or devices in operation.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "to comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

The various aspects discussed in this patent can be combined in order to provide additional advantages. Further, the person skilled in the art will understand that embodiments can be combined, and that also more than two embodiments can be combined.

## Claims

1. A microdermabrasion device (1) comprising a vacuum system (100) and a device tip (200), wherein the vacuum system (100) is in fluid communication with a channel inlet (120) at an inlet zone (1200) of the device tip (200), wherein the vacuum system (100) is configured to apply a vacuum to the inlet zone (1200), wherein the inlet zone (1200) further comprises an sensor (400) configured to measure a skin parameter of a part of a skin in the inlet zone (1200) and to provide a corresponding sensor signal, wherein the device tip (200) further comprises a microdermabrasion zone (1240) configured to abrade a part of said skin, and wherein the microdermabrasion device (1) further comprises a control unit (500) configured to control the vacuum as function of sensor signal information derived from the sensor signal and a predetermined relation between the sensor signal information and a vacuum setting.

2. The microdermabrasion device (1) according to claim 1, wherein the control unit (500) is configured to determine from the sensor signal information of the type of skin and control the vacuum as function of the skin type.

3. The microdermabrasion device (1) according to claim 1, wherein the control unit (500) is configured to determine from the sensor signal information a body zone and control the vacuum as function of the body zone.

4. The microdermabrasion device (1) according to any one of the preceding claims, wherein the control unit (500) is configured to maintain a constant protrusion of the skin into the inlet zone (1200).

5. The microdermabrasion device (1) according to any one of the preceding claims, wherein the vacuum system comprises a pump (105) and a bypass system (600) with a controllable vacuum leakage, wherein the control unit (500) is configured to control the vacuum applied to the inlet zone by controlling the controllable vacuum leakage.

6. The microdermabrasion device (1) according to any one of the preceding claims, further comprising a user interface (510) configured to allow a user select a user input parameter related to a strength of the vacuum, and wherein the control unit (500) is configured to control the vacuum as function of the user input parameter.

7. The microdermabrasion device (1) according to any one of the preceding claims, wherein the sensor (400) is selected from the group consisting of an optical sensor and an electrical conductivity sensor.

8. The microdermabrasion device (1) according to any one of the preceding claims, wherein the control unit (500) and the sensor (400) are configured to apply an optical measurement including one or more from the group consisting of (i) chromatic aberration, (ii) triangulation, (iii) light transmission, and (iv) light reflection, and wherein the sensor (400) is especially configured to measure one or more of protrusion depth of the skin in the inlet zone, the color of the skin, and the roughness of the skin.

9. The microdermabrasion device (1) according to any one of the preceding claims, further comprising a motion sensor (410), wherein the control unit (500) is configured to determine from one or more sensor signal information derived from the sensor signal and motion sensor signal information derived from a motion sensor signal from the motion sensor (410) one or more of a translation speed parameter and a translation direction parameter of the microdermabrasion device (1) and control the vacuum as function of one or more of said translation speed parameter and said translation direction parameter.

10. The microdermabrasion device (1) according to any one of the preceding claims, wherein the control unit (500) is configured to store one or more of (a) sensor signal information, and (b) treatment information, and wherein the control unit (500) is further configured to execute one or more of (i) controlling the vacuum as function of one or more of said sensor signal information and treatment information, and (ii) providing on a display information retrieved from one or more of said sensor signal information and treatment information.

11. The microdermabrasion device (1) according to any one of the preceding claims, further comprising an abrading material system (300) configured to provide in a gas flow abrading material (241) to the microdermabrasion zone (1240).

12. The microdermabrasion device (1) according to any one of the preceding claims, wherein the microdermabrasion zone (1240) comprises a microdermabrasion area (240) comprising immobilized abrading material (241).

13. The microdermabrasion device (1) according to any one of the preceding claims, wherein the control unit (500) is further configured to control the vacuum as function of one or more of a desired abrasion or effected abrasion.

14. The microdermabrasion device (1) according to any one of the preceding claims, wherein the channel inlet (120) is surrounded by a channel rim (220), and wherein the device tip (200) comprises a microdermabrasion area (240) configured remote from the channel inlet (120) with a recession (230) configured between the microdermabrasion area (240) and the channel rim (220), wherein the channel inlet (120) is perimetrically surrounded by the channel rim (220), and wherein the microdermabrasion area (240) perimetrically surrounds the channel rim (220), and having a vacuum area in the range of 10-400 mm², and wherein the device is configured to provide a negative pressure in the range of 5-80 kPa.

15. A non-therapeutic method for the controlled removal of at least part of the stratum corneum, the method comprising contacting the microdermabrasion device (1) as defined in any one of claims 1-14 with part of a skin and removing at least part of the stratum corneum while applying a vacuum to the to the inlet zone (1200).

## Patentansprüche

1. Mikrodermabrasionsvorrichtung (1), umfassend ein Vakuumsystem (100) und eine Vorrichtungsspitze (200), wobei das Vakuumsystem (100) mit einem Kanaleinlass (120) an einer Einlasszone (1200) der Vorrichtungsspitze (200) in Fluidkommunikation steht, wobei das Vakuumsystem (100) konfiguriert ist, ein Vakuum an die Einlasszone (1200) anzulegen, wobei die Einlasszone (1200) weiter einen Sensor (400) umfasst, der konfiguriert ist, einen Hautparameter eines Teils einer Haut in der Einlasszone (1200) zu messen und ein entsprechendes Sensorsignal bereitzustellen, wobei die Vorrichtungsspitze (200) weiter eine Mikrodermabrasionszone (1240) umfasst, die konfiguriert ist, einen Teil der Haut zu abradieren, und wobei die Mikrodermabrasionsvorrichtung (1) weiter eine Steuerungseinheit (500) umfasst, die konfiguriert ist, das Vakuum als Funktion einer von dem Sensorsignal stammenden Sensorsignalinformation und einer vorgegebenen Beziehung zwischen der Sensorsignalinformation und einer Vakuumeinstellung zu steuern.

2. Mikrodermabrasionsvorrichtung (1) nach Anspruch 1, wobei die Steuerungseinheit (500) konfiguriert ist, aus dem Sensorsignal Informationen über die Art der Haut zu ermitteln und das Vakuum als Funktion des Hauttyps zu steuern.

3. Mikrodermabrasionsvorrichtung (1) nach Anspruch 1, wobei die Steuerungseinheit (500) konfiguriert ist, aus dem Sensorsignal Informationen über eine Körperzone zu ermitteln und das Vakuum als Funktion der Körperzone zu steuern.

4. Mikrodermabrasionsvorrichtung (1) nach einem der vorstehenden Ansprüche, wobei die Steuerungseinheit (500) konfiguriert ist, eine konstante Protrusion der Haut in die Einlasszone (1200) aufrecht zu erhalten.

5. Mikrodermabrasionsvorrichtung (1) nach einem der vorstehenden Ansprüche, wobei das Vakuumsystem eine Pumpe (105) und ein Bypass-System (600) mit einem steuerbaren Vakuumleck umfasst, wobei die Steuerungseinheit (500) konfiguriert ist, das an die Einlasszone angelegte Vakuum durch Steuerung des steuerbaren Vakuumlecks zu steuern.

6. Mikrodermabrasionsvorrichtung (1) nach einem der vorstehenden Ansprüche, weiter umfassend eine Benutzeroberfläche (510), die konfiguriert ist, dem Benutzer die Auswahl eines mit einer Stärke des Vakuums zusammenhängenden Benutzereingabeparameters zu erlauben, und wobei die Steuerungseinheit (500) konfiguriert ist, das Vakuum als Funktion des Benutzereingabeparameters zu steuern.

7. Mikrodermabrasionsvorrichtung (1) nach einem der vorstehenden Ansprüche, wobei der Sensor (400) aus der Gruppe, bestehend aus einem optischen Sensor und einem elektrischen Leitfähigkeitssensor, ausgewählt ist.

8. Mikrodermabrasionsvorrichtung (1) nach einem der vorstehenden Ansprüche, wobei die Steuerungseinheit (500) und der Sensor (400) konfiguriert sind, eine optische Messung vorzunehmen, die eine oder mehrere aus der Gruppe, bestehend aus (i) chromatischer Aberration, (ii) Triangulation, (iii) Lichttransmission und (iv) Lichtreflexion, einschließt, und wobei der Sensor (400) insbesondere konfiguriert ist, eine oder mehrere der Protrusionstiefe der Haut in die Einlasszone, die Farbe der Haut und die Rauigkeit der Haut zu messen.

9. Mikrodermabrasionsvorrichtung (1) nach einem der vorstehenden Ansprüche, weiter umfassend einen Bewegungssensor (410), wobei die Steuerungseinheit (500) konfiguriert ist, aus einer oder mehreren von dem Sensorsignal stammenden Sensorsignalinformationen und von einem Bewegungssensorsignal des Bewegungssensors (410) stammenden Bewegungssensorsignalinformationen einen oder mehrere eines Übersetzungsgeschwindigkeitsparameters und eines Übersetzungsrichtungsparameters der Mikrodermabrasionsvorrichtung (1) zu ermitteln und das Vakuum als Funktion eines oder mehrerer des Übersetzungsgeschwindigkeitsparameters und des Übersetzungsrichtungsparameters zu steuern.

10. Mikrodermabrasionsvorrichtung (1) nach einem der vorstehenden Ansprüche, wobei die Steuerungseinheit (500) konfiguriert ist, eine oder mehrere (a) einer Sensorsignalinformation und (b) einer Behandlungsinformation zu speichern, und wobei die Steuerungseinheit (500) weiter konfiguriert ist, eines oder mehreres von Folgendem auszuführen: (i) Steuern des Vakuums als Funktion einer oder mehrerer der Sensorsignalinformation und der Behandlungsinformation und (ii) Bereitstellen von Informationen, die von einer oder mehreren der Sensorsignalinformation und der Behandlungsinformation abgerufen werden, auf einer Anzeige.

11. Mikrodermabrasionsvorrichtung (1) nach einem der vorstehenden Ansprüche, weiter umfassend ein Abrasionsmaterialsystem (300), das konfiguriert ist, der Mikrodermabrasionszone (1240) Abrasionsmaterial (241) in einem Gasstrom zur Verfügung zu stellen.

12. Mikrodermabrasionsvorrichtung (1) nach einem der vorstehenden Ansprüche, wobei die Mikrodermabrasionszone (1240) eine immobilisiertes Abrasionsmaterial (241) umfassende Mikrodermabrasionsfläche (240) umfasst.

13. Mikrodermabrasionsvorrichtung (1) nach einem der vorstehenden Ansprüche, wobei die Steuerungseinheit (500) weiter konfiguriert ist, das Vakuum als Funktion einer oder mehrerer einer erwünschten Abrasion oder einer durchgeführten Abrasion zu steuern.

14. Mikrodermabrasionsvorrichtung (1) nach einem der vorstehenden Ansprüche, wobei der Kanaleinlass (120) von einem Kanalrand (220) umschlossen ist und wobei die Vorrichtungsspitze (200) eine Mikrodermabrasionsfläche (240) umfasst, die entfernt von dem Kanaleinlass (120) konfiguriert ist, mit einer Vertiefung (230), die zwischen der Mikrodermabrasionsfläche (240) und dem Kanalrand (220) konfiguriert ist, wobei der Kanaleinlass (120) von dem Kanalrand (220) perimetrisch umschlossen ist, und wobei die Mikrodermabrasionsfläche (240) den Kanalrand (220) perimetrisch umschließt, und mit einer Vakuumfläche im Bereich von 10-400 mm², und wobei die Vorrichtung konfiguriert ist, einen Unterdruck im Bereich von 5-80 kPa bereitzustellen.

15. Nichttherapeutisches Verfahren zur gesteuerten Entfernung mindestens eines Teils des Stratum corneum, wobei das Verfahren das In-Kontakt-Bringen der Mikrodermabrasionsvorrichtung (1) nach einem der Ansprüche 1-14 mit einem Teil einer Haut und das Entfernen mindestens eines Teils des Stratum corneum beim Anlegen eines Vakuums an die Einlasszone (1200) umfasst.

## Revendications

1. Dispositif de microdermabrasion (1) comprenant un système de vide (100) et une pointe de dispositif (200), dans lequel le système de vide (100) est en communication fluidique avec une entrée de canal (120) au niveau d'une zone d'entrée (1200) de la pointe de dispositif (200), dans lequel le système de vide (100) est configuré pour appliquer un vide à la zone d'entrée (1200), dans lequel la zone d'entrée (1200) comprend en outre un capteur (400) configuré pour mesurer un paramètre de la peau d'une partie d'une peau dans la zone d'entrée (1200) et pour fournir un signal de capteur correspondant, dans lequel la pointe de dispositif (200) comprend en outre une zone de microdermabrasion (1240) configurée pour abraser une partie de ladite peau, et dans lequel le dispositif de microdermabrasion (1) comprend en outre une unité de régulation (500) configurée pour réguler le vide en fonction des informations du signal du capteur dérivées du signal du capteur et d'une relation prédéterminée entre les informations du signal du capteur et un réglage du vide.

2. Dispositif de microdermabrasion (1) selon la revendication 1, dans lequel l'unité de régulation (500) est configurée pour déterminer à partir du signal du capteur des informations sur le type de peau et réguler le vide en fonction du type de peau.

3. Dispositif de microdermabrasion (1) selon la revendication 1, dans lequel l'unité de régulation (500) est configurée pour déterminer à partir du signal du capteur des informations sur une zone corporelle et réguler le vide en fonction de la zone corporelle.

4. Dispositif de microdermabrasion (1) selon l'une quelconque des revendications précédentes, dans lequel l'unité de régulation (500) est configurée pour maintenir une partie saillante constante de la peau à l'intérieur de la zone d'entrée (1200).

5. Dispositif de microdermabrasion (1) selon l'une quelconque des revendications précédentes, dans lequel le système de vide comprend une pompe (105) et un système de dérivation (600) ayant une fuite de vide pouvant être régulée, dans lequel l'unité de régulation (500) est configurée pour réguler le vide appliqué à la zone d'entrée en régulant la fuite de vide pouvant être régulée.

6. Dispositif de microdermabrasion (1) selon l'une quelconque des revendications précédentes, comprenant en outre une interface utilisateur (510) configurée pour permettre à un utilisateur de sélectionner un paramètre d'entrée par l'utilisateur relatif à une intensité du vide, et dans lequel l'unité de régulation (500) est configurée pour réguler le vide en fonction du paramètre d'entrée par l'utilisateur.

7. Dispositif de microdermabrasion (1) selon l'une quelconque des revendications précédentes, dans lequel le capteur (400) est choisi dans le groupe constitué d'un capteur optique et d'un capteur de conductivité électrique.

8. Dispositif de microdermabrasion (1) selon l'une quelconque des revendications précédentes, dans lequel l'unité de régulation (500) et le capteur (400) sont configurés pour appliquer une mesure optique incluant une ou plusieurs du groupe constitué de (i) aberration chromatique, (ii) triangulation, (iii) transmission de la lumière, et (iv) réflexion lumineuse, et dans lequel le capteur (400) est particulièrement configuré pour mesurer une ou plusieurs de la profondeur de partie saillante de la peau dans la zone d'entrée, de la couleur de la peau, et de la rugosité de la peau.

9. Dispositif de microdermabrasion (1) selon l'une quelconque des revendications précédentes, comprenant en outre un capteur de mouvement (410), dans lequel l'unité de régulation (500) est configurée pour déterminer à partir d'une ou plusieurs informations du signal du capteur dérivées du signal du capteur et informations du signal du capteur de mouvement dérivées d'un signal de capteur de mouvement à partir du capteur de mouvement (410) un ou plusieurs d'un paramètre de vitesse de translation et d'un paramètre de direction de translation du dispositif de microdermabrasion (1) et réguler le vide en fonction d'un ou plusieurs dudit paramètre de vitesse de translation et dudit paramètre de direction de translation.

10. Dispositif de microdermabrasion (1) selon l'une quelconque des revendications précédentes, dans lequel l'unité de régulation (500) est configurée pour stocker une ou plusieurs de (a) informations du signal du capteur, et (b) informations de traitement, et dans lequel l'unité de régulation (500) est en outre configurée pour exécuter une ou plusieurs de (i) régulation du vide en fonction d'une ou plusieurs desdites informations du signal du capteur et informations de traitement, et (ii) fourniture sur un affichage d'informations récupérées à partir d'une ou plusieurs desdites informations du signal du capteur et informations de traitement.

11. Dispositif de microdermabrasion (1) selon l'une quelconque des revendications précédentes, comprenant en outre un système de matériau d'abrasion (300) configuré pour fournir un matériau d'abrasion à courant gazeux (241) dans la zone de microdermabrasion (1240).

12. Dispositif de microdermabrasion (1) selon l'une quelconque des revendications précédentes, dans lequel la zone de microdermabrasion (1240) comprend une surface de microdermabrasion (240) comprenant un matériau d'abrasion immobilisé (241).

13. Dispositif de microdermabrasion (1) selon l'une quelconque des revendications précédentes, dans lequel l'unité de régulation (500) est en outre configurée pour réguler le vide en fonction d'une ou plusieurs d'une abrasion souhaitée ou d'une abrasion effectuée.

14. Dispositif de microdermabrasion (1) selon l'une quelconque des revendications précédentes, dans lequel l'entrée de canal (120) est entourée par une bordure de canal (220), et dans lequel la pointe de dispositif (200) comprend une surface de microdermabrasion (240) configurée à distance de l'entrée de canal (120) avec un évidement (230) configuré entre la surface de microdermabrasion (240) et la bordure de canal (220), dans lequel l'entrée de canal (120) est entourée de manière périmétrique par la bordure de canal (220), et dans lequel la surface de microdermabrasion (240) entoure de manière périmétrique la bordure de canal (220), et ayant une surface de vide dans la plage de 10-400 mm², et dans lequel le dispositif est configuré pour fournir une pression négative dans la plage de 5-80 kPa.

15. Procédé non thérapeutique pour l'élimination régulée d'au moins une partie de la couche cornée, le procédé comprenant la mise en contact du dispositif de microdermabrasion (1) selon l'une quelconque des revendications 1-14 avec une partie d'une peau et l'élimination d'au moins une partie de la couche cornée tout en appliquant un vide à la zone d'entrée (1200).
